# EUROPEAN PATENT SPECIFICATION

(11) **EP 1 530 463 B1**
(45) Date of publication and mention of the grant of the patent: **14.04.2010**
(21) Application number: 02774591.8
(22) Date of filing: 10.09.2002
(51) Int. Cl.: A61K 31/198, A61K 31/191, A61K 31/194, A61P 9/00

(54) **L-ARGININE CONTAINING PHARMACEUTICAL COMPOSITION**
PHARMAZEUTISCHE ZUSAMMENSETZUNG ENTHALTEND L-ARGININ
COMPOSITION PHARMACEUTIQUE CONTENANT DE LA L-ARGININE

(30) Priority: 22.08.2002 WO PCT/EP02/09395
(43) Date of publication of application: 18.05.2005
(73) Proprietor: Vasopharm Biotech GmbH, 97076 Würzburg (DE); Pascoe Pharmazeutische Präparate GmbH, 35394 Giessen (DE)
(72) Inventor: SCHMIDT, Harald, 35392 Giessen (DE); WINGLER, Kirstin, Hughesdate, Vic 3166 (AU); KOMESKER, Georg, 35578 Wetzlar (DE)
(74) Representative: Viering, Jentschura & Partner
(86) International application number: PCT/EP2002/010147
(87) International publication number: WO 2004/017955

(56) References cited:
- EP-A- 0 908 182
- EP-A- 1 163 904
- US-A- 6 063 432
- US-A1- 2002 091 156
- HELLER, REGINE (1) ET AL: "L- ascorbic acid potentiates endothelial nitric oxide synthesis via a chemical stabilization of tetrahydrobiopterin." JOURNAL OF BIOLOGICAL CHEMISTRY, (JANUARY 5, 2001) VOL. 276, NO. 1, PP. 40-47. PRINT., XP001121133

## Description

The present invention refers to a pharmaceutical composition containing L-Arginine, to a method for producing the pharmaceutical composition as well as to various medical and therapeutic uses of the pharmaceutical composition.

The physiological role of L-arginine, a semi-essential amino acid, has been an area of active study for many years. L-arginine is known to be important in a range of biological, biochemical and metabolic processes. It has also attracted considerable attention because of its therapeutic and prophylactic potency and has been introduced into clinical practice for a range of clinical conditions such as those relating to traumatized patients, patients with sepsis and patients with malignant diseases. An overview of present biomedical investigations and possible applications is given by Eremin (Ed.) in "L-Arginine: Biological Aspects and Clinical Applications, Springer-Verlag, Heidelberg, Germany, 1997, ISBN: 3-540-61171-7.

An area which has become the subject of intensive research recently is the involvement of L-arginine in the treatment of disorders which are associated with a disturbed nitric oxide (NO) level. Nitric oxide has various physiological functions. It has, for example, a relaxing effect on the smooth vascular musculature and by so doing is substantially involved in the regulation of the blood pressure. NO also controls blood clotting via inhibition of platelet aggregation. Accordingly, a disturbed NO balance or level results in serious disorders and damages such as septic shock, arterial high blood pressure, hemostasis disorders, coronary heart and vascular diseases and erectile dysfunction. L-Arginine has attracted interest in this conjunction because endogenous NO is formed from arginine by at least three different NO synthase isoenzymes (see, for example, Kerwin, J.F., Jr. and Heller, M. Med. Res. Rev. 14 (1994), 23; MacAllister, R. and Benjamin, N. in Eremin, supra, 79).

Several approaches for administration of arginine both in clinical studies and practical use are known. In clinical studies L-arginine is, for instance, dissolved in saline buffer and then administered by infusion (see Stroes, E., J. Clin. Invest. 99, (1997) 41-46, for example). US Patent 5,217,997 which refers to the use of L-arginine for the treatment of hypertension (which is characterized by persistently high blood pressure) discloses that L-arginine or any salt of L-arginine can be administered to a patient using any suitable pharmaceutically acceptable inter carrier material. US patents 5,891,459 and 5,428,070 disclose use of L-arginine as dietary supplement. According to these patents such a supplement is a pharmaceutically acceptable salt such as the hydrochloride salt or the glutamate salt of L-arginine. US patent 5,428,070 further teaches to incorporate L-arginine in a food such as a health bar, e.g. granola, other grains or fruits bars. US patent 6,063,432 describes an arginine containing health bar formulation which contains L-arginine in combination with, for example, fruit as paste and solids of carbohydrates, and dietary fiber to form an organoleptically acceptable food supplement. A further approach is known from US patent 6,117,872 which discloses a formulation containing L-arginine and additives such as vitamins A, C and E or factors, for example biopterins, which are said to prolong the half-life of endothelial-derived nitric oxide, i.e. to further support the NO production initiated by the supplementation with endogenous L-arginine. According to US 6,117,872, the composition disclosed can be included in a liquid food substance such as a soft drink. Finally, a powder mixture called HeartBar® Orange Drink is sold by the company Cooke Pharma Inc. According to the label of the product, this powder mixture contains among various ingredients L-arginine, citric acid, maltodextrine, ascorbic acid and soy isoflavone extract (see also http:www.cookepharma.com/productline.asp). Judging from the nutrition facts on the quantity of ascorbic acid and the recommended intake of L-arginine, a serving of 9 grams of the powder mixture contains 3 grams of L-arginine and about 250 mg (420 % of the recommended daily FDA value) ascorbic acid. Finally, the German Offenlegungsschrift DE 198 00 812 describes an oral formulation which comprises L-arginine hydrochloride, an effervescent agent and an acid. According to DE 198 00 812, the effervescent agent can be any one of the conventional CO₂ generating agents, preferably, sodium hydrogen carbonate, calcium carbonate or mixtures thereof. The presence of this effervescent agent is considered to be essential for improving the taste of the oral formulation.

In view of the various applications and ways of administration of L-arginine, e.g. administration by infusion, oral administration in form of an edible or a drinkable formulation, it would be desirable to have a pharmaceutical composition that can be employed in manifold ways. It is thus an object of the present invention to provide a pharmaceutical composition containing L-arginine that is suitable for many ways of administration.

This problem is solved by the pharmaceutical composition having the features of the claims.

Such a composition is an L-arginine containing pharmaceutical composition, wherein the composition contains L-arginine or a pharmaceutically acceptable salt thereof in solid form as well as a pharmaceutically acceptable organic acid, such as citric acid, in solid form, and, wherein the quantity of the organic acid present in the composition is chosen such that the molar ratio of the acid functionalities of the organic acid to one or both of the two basic centers of L-arginine ranges from 0.5 to 1.5.

In other words, the present invention is based on the finding that neutralizing one or both of the two basic centers of arginine by use of an organic acid yields a solid pharmaceutical composition containing L-arginine that has several advantages. First, such a pharmaceutical composition can be obtained in an easy manner, for example, by mixing solid L-arginine with a suitable pharmaceutically acceptable acid in a certain molar ratio. Second, the pharmaceutical composition is easy to store and is stable over a long period of time.

A composition composed of L-arginine and an organic acid, such as citric acid, can be used for infusion, by simply dissolving the composition in water or saline. When doing so, the organic acid and the quantity of water can be used such that the pH of the resulting infusion solution is in the physiological range, for example, similar to the pH of blood which ranges from 7.3 to 7.4 and the osmotic pressure of the solution is in an acceptable range.

The pharmaceutical composition can also be used for the preparation of medical food products, for example, as basis for an instant drink. In this conjunction, it should be noted that the pharmaceutical composition can be formulated to contain high concentrations of L-arginine and thus facilitates administration of high doses (up to 6 grams per day or more) in a convenient manner and also an acceptable taste, if used as an instant drink.

The term L-arginine as used in the present invention includes all forms which provide L-arginine as the pharmaceutically active ingredient. L-arginine can also employed in peptidic form, for example as dipeptide (L-arginyl-L-arginine), tripeptide (Arg)₃ or tetrapeptide (Arg)₄. Suitable oligopeptides include oligopeptides of from 2 to 30, generally 2 to 20 and preferably 2 to 10 monomer residues. The use of longer peptides is, however, possible. Arginine can also be employed in the form of poly-L-arginine having a molecular weight of 5.000 to 15.000 or of 15.000 to 70.000, for example. Such polymeric forms are available from Sigma-Aldrich GmbH, Germany, for instance. The arginine-containing oligo- and polypeptides used in the present invention do not necessarily only contain L-arginine. They can also contain other amino acids such as lysine, glycine, ornithine or other amino acids and other building blocks, as long as L-arginine is deliberated upon administrating the composition of the invention and as long as the amino acid(s) or other building blocks are pharmaceutical acceptable.

L-arginine can also be used in the form of prodrugs or galenic retard forms from which L-arginine can be released. Such prodrugs or retard forms are preferably used when a sustained or controlled release of L-arginine is intended. Examples of suitable prodrugs and retard forms are microencapsulated L-arginine, ornithine and peptides that contain L-arginine and any other formulation that contains arginine precursors and any other galenic formulation that results in sustained release of L-arginine.

It is also possible to use a pharmaceutically acceptable salt of L-arginine as a source of L-arginine. In these salts, L-arginine is present as positively charged cation and it this associated with a negatively charged counter-ion, i.e. an anion. As will explained in more detail below, this anion is preferably an anion of a strong inorganic acid such as but not restricted to hydrochloric acid, sulfuric acid, nitric acid or phosphoric acid. Accordingly, preferred embodiments of the pharmaceutically acceptable salt of L-arginine are e.g. L-arginine hydrochloride, L-arginine sulfate, L-arginine nitrate, L-arginine phosphate or mixtures thereof. It is also possible to use salts L-arginine forms with pharmaceutically acceptable organic acids such as acetic acids, ascorbic acid, lactid acid, tartaric acid, malic acid, oxalic acid or glutamic acid. In this respect, it should be noted that the acid used for formation of a pharmaceutically acceptable salt of arginine has to be distinguished from the organic acid that is used for neutralizing one of the two basic centers of arginine. The latter organic acid is present in the composition of the invention in any case, whereas the acid used for formation of an pharmaceutical acceptable salt of L-arginine is an optional component which can be "indirectly" present in the composition of the invention when a suitable anion for formation of an salt of L-arginine is desired.

L-arginine can also be present as a mixture of any of the above-mentioned forms, e.g. as a mixture of a pharmaceutically acceptable salt and an oligomer of L-arginine alone or in combination with other amino acids.

As noted above, the quantity of the organic acid present in the composition of the invention is chosen such that the molar ratio of the acid functionalities of the organic acid to one or both basic centers of L-arginine ranges from 0.5 to 1.5. By so doing, one or both of these basic centers of arginine will be neutralized once the solid composition of the invention has been dissolved. The term "basic center of arginine" relates to the two basic moieties present in this amino acid, i.e. to the δ-guanido group of the side chain of arginine and the amino function at the α-carbon atom.

The term "acid functionality" means an acid group which can donate a proton (H⁺-ion) in solution to a corresponding acceptor such as an hydroxide-ion in an acid-base reaction. Accordingly, the quantity of the organic acid present in the composition depends on the number of acid moieties/functionalities which are available in the organic acid for reaction (with a base or a proton acceptor). An acid such as ascorbic acid or lactic acid, that has one acid group, will thus be present in the composition in an quantity of 0.5 to 1.5 moles related to the chosen quantity of L-arginine in order to yield a molar ratio of the acid functionalites to one of the two basic centers of arginine in the range of 0.5 to 1.5. If an equimolar ratio (1:1) of the acid functionalities and one of the two basic centers of L-arginine is wanted in the composition, ascorbic acid and L-arginine are then present in equimolar quantities. If an acid having three acid functionalities or acid groups such as citric acid is utilized in the composition of the invention, only a third of the molar quantity of L-arginine has to be used in case an equimolar ratio of the acid functionalities and one of the two basic centers of L-arginine is desired. In addition, the second basic center of L-arginine can be neutralized in an analogous way.

The exact molar ratio between the organic acid and L-arginine varies dependent on the specific use of the composition of the invention, the form in which the L-arginine is employed, and also on the "strength", i.e. the pKₐ-value, of the organic acid. The forms which can be chosen for incorporation of L-arginine are also referred to any delivery forms of L-arginine in the following.

For example, if L-arginine is employed in the form of the pure amino acid, its guanidino group will react as strong base after being dissolved. If the pharmaceutical composition of the invention is intended to be used for infusion, the resulting infusion should have a nearly neutral pH of about 7.0 to 7.4. Due to the strong basic behavior of the guanidino group (which is reflected by a pKₐ of 12.48), an organic acid such as ascorbic acid with a pKₐ of 4.17 or lactic acid with a pKₐ of 3.79 will deliver (almost) all its protons in an corresponding acid-base reaction. Accordingly, an essentially equimolar quantity of acid functionalities of an organic acid such as ascorbic acid will be added in order to provide a sufficient amount of H⁺-ions for neutralization of the OH⁻-ions created by the basic reaction of the guanidino group. This in turn will preferably result in a substantially neutral pH of the solution formed. If the organic acid should additionally provide a buffering effect, an amount higher of the organic acid would be added, for example an 1.3 molar excess of acid functionalities related to one of the two basic groups of arginine might be desirable. If the composition is to serve as a basis of an instant drink, a more acidic pH can be tolerable or even desired, if for example, the taste of the drink would be improved by a lower pH value. In this case, a stronger organic acid might be used or the amount of the organic acid might be increased. Increasing the amount of the organic acid is also a way of increasing the osmolarity of a resulting solution. The exact molar ratios can thus be determined empirically, depending on the particular application. A corresponding approach would be taken, if an oligopeptide, e.g. arginyl-arginine, which shows a similar basic behavior would be used in the pharmaceutical composition.

When arginine is used in the form of a salt such as the hydrochloride, these salts usually react slightly acidic in an aqueous solution. In this case, addition of an organic acid such as ascorbic acid or citric acid serves more to increase the buffering capacity or to increase the osmolarity of a resulting solution than to react with hydroxide-ions and, if the composition is used as a medical food to improve the taste of L-arginine or its salts. However, if a salt of arginine that contains an anion that functions as a base (e.g. an acetate-ion), the organic acid can serve as a neutralizing agent also in this case. If such a basic reaction of the anion of the salt of arginine is to be avoided then inorganic acids are preferably used for formation of the arginine salt. Again, the exact molar ratio of the organic acid used can vary from case to case and can be optimized empirically.

From the above it gets evident that the composition of the present invention provides an easy way to adapt a pharmaceutical composition for various different applications.

In a preferred embodiment of the invention, the quantity of the organic acid is chosen such that the molar ratio of the acid functionalities of the organic acid to one of the two basic centers of L-arginine ranges from 0.7 to 1.4, more preferably from 0.8 or 0.85 to 1.3. Even more preferred is a molar ratio of the acid functionalities of the organic acid to one of the two basic centers of L-arginine in the range from 0.9 to 1.2. In an embodiment, in which the composition of the invention is used as a (instant) drink, an quantity of the organic acid corresponding to a molar ratio of 1.1 acid functionalities to the amount of one of the two basic centers of arginine is the most preferred value.

In a further preferred embodiment, the pharmaceutically acceptable salt of L-arginine is L-arginine hydrochloride.

The organic acids can generally be any suitable pharmaceutically acceptable organic acid which is present in solid form (at room temperature). In preferred embodiments, the organic acid is citric acid, lactic acid, maleic acid acid, fumaric acid, malic, tartaric acid whereby citric acid is the most preferred These organic acids can also be used as mixtures as long as the organic acids are compatible with each other. In preferred embodiments, the composition does not contain any further naturally occurring amino acid besides L-arginine.

The pharmaceutical composition of the invention can exclusively consist of L-arginine and the pharmaceutically acceptable organic acids. In a preferred embodiment, however, it contains further compounds or additives. Preferred compounds are those which increase the formation of NO from endogenous arginine. Preferred additives of this kind are cofactors of NO synthases such as (6R)-5,6,7,8-tetrahydro-L-biopterin (to which will also be referred to as BH4 in the following), (6R)-5,6,7,8-tetrahydro-L-biopterin-hydrochlorid or 6-methyltetrahydropterin, or sepiapterin from which BH4 is obtained in vivo and other precursors of BH4 or any salts of BH4, methyltetrahydropterin or sepiapterin, e.g. ascorbates (all of which will be included in the abbreviation BH4 in the following). These additives are preferably present in the forms of appropriate salts. The use of a salt such as the dihydrochloride is in particular preferred for BH4 because the salt form reduces the susceptibility of BH4 to oxidation. As found by the present inventors, a further stabilization of BH4 can be achieved by use of L-arginine hydrochloride. BH4 can be present as the physiologically active stereoisomer 6R-5,6,7,8-tetrahydrobiopterin-L-biopterin or as racemic mixture with 6S-5,6,7,8-tetrahydrobiopterin-L-biopterin.

Other compounds which can be incorporated into the composition disclosed here are vitamins and antioxidants and phytochemicals such as but not restricted to flavonoids, isoflavonoids, carotenoids, catechins, tannins other than vitamin C (ascorbic acid). Such compounds can either further enhance the therapeutic activity of L-arginine or may have an additional own physiologically advantageous effect. Vitamins that may be incorporated include but are not restricted to vitamin A, B₆ and B₁₂, D, E, and K, thiamin, riboflavin, folic acid, pantothenic acid, or biotin. Unsaturated fatty acids such as ω-3 unsaturated fatty acids can also be included into the composition of the present invention. Other compounds that may be comprised in the composition are cysteine, glutathione, carnitine or coenzyme Q. Minerals and trace metals are a further class of compounds that can be added. Minerals and trace elements of interest include but are not restricted to calcium, magnesium, iodine, iron, zinc, selenium, copper, manganese, molybdenum or chromium in a compatible manner.

In preferred embodiment of the composition, L-arginine is included as arginine-hydrochloride and the organic acid is chosen from ascorbic acid, citric acid and lactic acid. Particularly preferred is a composition which comprises L-arginine hydrochloride and ascorbic acid or which consists of these two compounds only.

The pharmaceutical composition is suitable for various ways of administration, preferably for parenteral administration by injection or infusion or for oral or enteral administration and also for topical administration. For parenteral administration the composition consists either of the delivery form of L-arginine and the pharmaceutically acceptable salt or peptide and an organic acid or it also contains an one or more of the other compounds mentioned above. As already noted, the formulation suitable for parenteral administration can be obtained by dissolving the solid composition in water or saline. For topical application the composition can be incorporated into a liquid, a lotion, an ointment or a crème, for instance. In accordance with these uses and ways of administration, a generally preferred embodiment of the composition is free of an effervescent agent, i.e. a CO₂-generating agent. This does not only apply for the composition employed for enteral or topical application, where it is directly evident that the presence of such a CO₂ generating agent is undesired, but also for oral administration (cf. Examples).

For its use, the composition of the invention may be formulated as a solid mixture of arginine with the organic acid and optionally further components. In an alternative embodiment the composition is in the form of a two-part kit, i.e. a kit comprising two separate containers. One container includes the respective form of arginine, alone or in admixture with further compounds such as but not restricted to BH4, sepiapterin or methyltetrahydropterin or any other of the above mentioned additives alone or in combination. The other container includes the organic acid. For preparing a formulation which is used for infusion, the content of the two containers are either first mixed and then dissolved together or they are sequentially dissolved in the same solution or in two different solutions which are then combined.

In preferred embodiments for oral or enteral administration, the composition can be used as a food supplement. It can be included into a food such as but not restricted to a bar or ingredient in a common food, e.g. flour, pasta or other foods or it can be utilized in a drink. In a preferred embodiment, the composition of the invention is an instant formulation which can be used for the preparation of a drink. In this embodiment, the composition further comprises a sweetener, a flavoring agent or a mixture thereof. In principle every known sweetener can be used. Compounds having a low calorie content and/or a low carious potential are preferred. Examples of suitable sweeteners are e.g. but not restricted to oligofructose/inuline, fructose or sugar replacement compounds such as isomalt®, sucralose, aspartame, cyclamate, saccharine or acesulfam-K which are also known as non nutritive sweeteners. In a preferred embodiment of the composition, oligofructose/inuline is used. Sugar alcohols such as sorbitol or mannitol may also be used as sweetener. In this respect it is noted that compounds such as polymeric fructose, e.g. inuline or oligofructose do not only act as sweetener but also as dietary fiber. By using inuline or oligofructose as a sweetener, the composition obtains prebiotic properties, which is an additional advantage of using inuline or oligofructose as a sweetener.

Any suitable flavoring agent can be included in the composition of the invention. Exemplary fruit flavors are but not restricted to orange flavor, lemon flavor, cherry flavor, strawberry flavor. Other food flavors such as vanilla or chocolate flavor can, of course, also be used.

For oral administration the composition may also be formulated as, tablets, film- or sugar coated tablets, capsules or effervescent tablet. In a preferred embodiment for oral administration, the composition does not contain any CO₂ generating agent such as sodium hydrogen carbonate.

The amount of L-arginine in the composition of the invention can vary in a large range. It depends, for example, on the intended use and on the molar ratio in which arginine and the organic acid are present. Usually, a unit dosage composition contains an amount that enables to cover at least the daily need of L-arginine. An unit dosage formulation thus usually contains 1 to 25 grams, more preferably 3 to 15 grams of L-arginine.

When used as a dietary supplement, for example, as an instant drink, the composition is formulated such that a quantity from preferably 3 to 9 grams, or 3 to 6 grams can be taken up by an individual in one, two or more servings. The exact amounts can vary and depend, for instance, on the type of organic acid that is used. In case an organic acid with one acid group such as ascorbic acid is used, an instant formulation according to the invention typically comprises 20 to 60 weight percent L-arginine, up to 30 weight percent of a sweetening agent, up to 25 weight percent flavoring agents, and the balance organic acid. If citric acid which has three acid groups is used as organic acid, then the amount of L-arginine might be increased because less organic acid is necessary to achieve the molar ratio of the acid functionalities and one of the two basic centers of arginine of from 0.5 to 1.5. In addition the drink can contain L-arginine containing peptides instead of L-arginine. It can also contain any other ingredients listed above.

The composition of the invention can in general be used for all therapeutic applications in which administration of L-arginine is beneficial to a patient, i.e. it can be used for the treatment of a disorder or a disease or it can be taken prophylactically, i.e. in order to avoid the development or re-occurrence of a disease or disorder.

A preferred therapeutic application is the treatment of endothelial dysfunction and diseases and disorders that are caused by endothelial dysfunction. The term "endothelial dysfunction" is used herein in accordance with its regular meaning as given, for example, in Nachrichten aus der Chemie Vol. 49, pages 1411-1415, Dec. 2001: Endotheliale Dysfunktion: Vom Markern zu Medikamenten. Thus, endothelial dysfunction means that the endothelium of the vessels cannot function in a normal way anymore, e.g. due to reduced bioavailability of NO.

Examples of such diseases or disorders which are associated with endothelial dysfunction are e.g. hypercholesterolemia, hypertension, hypoxia, endothelial damage due to mechanical und chemical noxia, peripherial arterial disease and cardiovascular diseases such as atherosclerosis, heart disease, coronary heart disease, diabetic micro- and macroangiopathy, heart failure, stroke, angina pectoris and other diseases which rely on or are caused by endothelial dysfunction.

The compositions can also be used for the treatment of diseases that are not associated with endothelial dysfunction. Examples of such diseases which may be treated by use of the pharmaceutical composition of the invention are e.g. but not restricted to cancer, osteoporosis, diabetes mellitus, erectile dysfunction, chronic pain and renal failure. The composition can also be used for enhancement of wound healing, treatment of infected wounds or eczemae of all kinds, in particular skin eczemae. In this case the composition is preferably formulated to be suitable for topical administration, for instance included into a lotion or an ointment, however, can also be administered systemically, for instance using a drinkable composition as disclosed here.

In accordance with the above disclosure, the present invention also refers to a method of treating a disease or disorder in a mammal, comprising the step of administering an L-arginine containing pharmaceutical composition of the invention to a mammal in need thereof. In generally, every mammal can be treated with the inventive compositions. Preferred mammals are humans, monkeys, rats, cats, dogs and cows, pigs or any other animals such us e.g. birds.

The invention also refers to a method for preparing a pharmaceutical composition containing L-arginine. This method comprises mixing L-arginine or a pharmaceutically acceptable salt thereof in solid form with an organic acid in solid form, wherein the quantity of the organic acid present in the composition is chosen such that the molar ratio of the acid functionalities of the organic acid to one or both of the two basic centers of L-arginine ranges from 0.5 to 1.5.

The following examples; are not within the scope of the claims

### Example 1: Preparation of a pharmaceutical composition

A pharmaceutical composition was prepared by mixing 50,0 grams (0.23734 mole, calculated using a mole weight of 210.67 g) of L-arginine-hydrochloride with 42,7 grams (0,242304 mole) ascorbic acid. Thus, the organic acid is present in a quantity corresponding to a ratio of the acid functionalities to one of the two basic groups of L-arginine of 1.02. This composition is suitable for intravenous administration by dissolving the composition in water.

### Example 2: Preparation of an instant formulation

a) 50,0 g (0.23734 mole) of L-arginine-hydrochloride were mixed with 42,7 g (0,242304 mole) ascorbic acid, 2.0 g orange flavor and 10,3 g inuline/oligofructose. This yielded a composition of the invention which contains approximately 2 g L-arginine in a serving (one teaspoon) of 5 grams. A serving of approximately one teaspoon was dissolved in water and the resulting drink was drunk by test persons. According to the test persons the taste was not acceptable.
b) 50,0 g (0.23734 mole) of L-arginine-hydrochloride were mixed with 44,7 g (0,253842 mole) ascorbic acid, 3.0 g orange flavor and 15,3 g inuline/oligofructose. This yielded a composition of the invention which contains 1.84 g L-arginine in a serving (one teaspoon) of 5 grams. A serving of approximately one teaspoon was dissolved in water and the resulting drink was drunk by test persons. According to the test person, the taste of the drink was better compared to the taste of the drink of Example 2a).
c) 50,0 g (0.23734 mole) of L-arginine-hydrochloride were mixed with 44,7 g (0,253842 mole) ascorbic acid, 2.5 g orange flavor and 20,8 g inuline/oligofructose. This yielded a composition of the invention which contains 1,84 g L-arginine in a serving (one teaspoon) of 5 grams. A serving of one teaspoon was dissolved in water and the resulting drink was drunk by test persons. According to the test persons, the taste of the drink was the best compared to the taste of the drinks of Example 2a, 2b and suitable for daily use. Accordingly, an quantity of 7 grams arginine can be administered in a convenient way by two servings of each 10 grams (two teaspoons) of a composition of the present invention.

## Claims

1. L-Arginine containing pharmaceutical composition, wherein the composition contains L-arginine or a pharmaceutically acceptable salt thereof in solid form as well as citric acid in solid form, wherein the composition is free of a CO₂ generating agent and, wherein the quantity of the citric acid present in the composition is chosen such that the molar ratio of the acid functionalities of the organic acid to one or both of the two basic centers of L-arginine ranges from 0.5 to 1.5.

2. Pharmaceutical composition according to claim 1, wherein the molar ratio of the acid functionalities of the organic acid to one of the two basic centers of L-arginine ranges from 0.8 to 1.3.

3. Pharmaceutical composition according to claim 1 or 2, wherein the molar ratio of the acid functionalities of the organic acid to one of the two basic centers of L-arginine ranges from 0.9 to 1.2.

4. Pharmaceutical composition according to any of claims 1 to 3, wherein the pharmaceutically acceptable salt is L-arginine hydrochloride or L-arginine ascorbate.

5. Pharmaceutical composition according to any of claims I to 4, wherein L-arginine is present as oligomer of L-arginine or poly-Larginine, or an oligomer containing L-arginine and other amino acids.

6. Pharmaceutical composition according to any of claims 1 to 5,wherein the composition further contains (6R)-5,6,7,8-tetrahydro-L-biopterin, (6R)-5,6,7,8-tetrahydro-L-biopterin-hydrochlorid, or 6- methyltetrahydropterin, or sepiapterin or a mixture thereof, or any salts of (6R)-5,6,7,8-tetrahydro-L-biopterin, methyltetrahydropterin or sepiapterin or a mixture thereof.

7. Pharmaceutical composition according to any of claims 1 to 6, wherein the composition is suitable for oral, enteral, parenteral or topical administration.

8. Pharmaceutical composition according to claim 7, wherein the composition further comprises a sweetener, a flavoring agent or a mixture thereof.

## Patentansprüche

1. L-Arginin-haltige pharmazeutische Zusammensetzung, wobei die Zusammensetzung L-Arginin oder ein pharmazeutisch annehmbares Salz davon in fester Form als auch Zitronensäure in fester Form enthält, wobei die Zusammensetzung frei von einem CO₂ bildenden Mittel ist und wobei die Menge der in der Zusammensetzung vorliegenden Zitronensäure so gewählt ist, dass das molare Verhältnis der Säurefunktionalitäten der organischen Säure zu einem oder beiden basischen Zentren von L-Arginin von 0.5 bis 1.5 reicht.

2. Pharmazeutische Zusammensetzung gemäß Anspruch 1, wobei das molare Verhältnis der Säurefunktionalitäten der organischen Säure zu einem der beiden Zentren des L-Arginins von 0.8 bis 1.3 reicht.

3. Pharmazeutische Zusammensetzung gemäß Anspruch 1 oder 2, wobei das molare Verhältnis der Säurefunktionalitäten der organischen Säure zu einem der beiden basischen Zentren des L-Arginins von 0.9 bis 1.2 reicht.

4. Pharmazeutische Zusammensetzung gemäß einem der Ansprüche 1 bis 3, wobei das pharmazeutisch annehmbare Salz L-Argininhydrochlorid oder L-Argininascorbat ist.

5. Pharmazeutische Zusammensetzung gemäß einem der Ansprüche 1 bis 4, wobei L-Arginin als Oligomer von L-Arginin oder Poly-L-Arginin oder als ein Oligomer, das L-Arginin und andere Aminosäuren enthält, vorliegt.

6. Pharmazeutische Zusammensetzung gemäß einem der Ansprüche 1 bis 5, wobei die Zusammensetzung weiter (6R)-5,6,7,8-Tetrahydro-L-biopterin, (6R)-5,6,7,8-Tetrahydro-L-biopterinhydrochlorid oder 6-Methyltetrahydropterin oder Sepiapterin oder eine Mischung davon oder irgendein Salz von (6R)-5,6,7,8-Tetrahydro-L-biopterin, Methyltetrahydropterin oder Sepiapterin oder eine Mischung davon enthält.

7. Pharmazeutische Zusammensetzung gemäß einem der Ansprüche 1 bis 6, wobei die Zusammensetzung für orale, enterale, parenterale oder topische Verabreichung geeignet ist.

8. Pharmazeutische Zusammensetzung gemäß Anspruch 7, wobei die Zusammensetzung weiter ein Süßungsmittel, einen Aromastoff oder eine Mischung davon umfasst.

## Revendications

1. Composition pharmaceutique contenant de la L-arginine, laquelle composition contient de la L-arginine ou un sel pharmaceutiquement acceptable de celle-ci sous forme solide ainsi que de l'acide citrique sous forme solide, laquelle composition est exempte d'agent générant du CO₂ et, où la quantité de l'acide citrique présent dans la composition est choisie de sorte que le rapport molaire des fonctionnalités acides de l'acide organique par rapport à un ou l'ensemble de deux centres basiques de la L-arginine est dans le domaine de 0,5 à 1,5.

2. Composition pharmaceutique selon la revendication 1, dans laquelle le rapport molaire des fonctionnalités acides de l'acide organique par rapport à l'un des deux centres basiques de la L-arginine est dans le domaine de 0,8 à 1,3.

3. Composition pharmaceutique selon la revendication 1 ou 2, dans laquelle le rapport molaire des fonctionnalités acides de l'acide organique par rapport à l'un des deux centres basiques de la L-arginine est dans le domaine de 0,9 à 1,2.

4. Composition pharmaceutique selon l'une quelconque des revendications 1 à 3, dans laquelle le sel pharmaceutiquement acceptable est un chlorhydrate de L-arginine ou un ascorbate de L-arginine

5. Composition pharmaceutique selon l'une quelconque des revendications 1 à 4, dans laquelle la L-arginine est présente en tant qu'oligomère de L-arginine ou poly-L-arginine, ou un oligomère contenant la L-arginine et d'autres acides aminés.

6. Composition pharmaceutique selon l'une quelconque des revendications 1 à 5, laquelle composition contient en outre la (6R)-5,6,7,8-tétrahydro-L-bioptérine, le chlorhydrate de (6R)-5,6,7,8-tétrahydro-L-bioptérine, ou la 6-méthyltétrahydroptérine, ou sépiaptérine ou un mélange de ceux-ci, ou un sel quelconque de la (6R)-5,6,7,8-tétrahydro-L-bioptérine, la méthyltétrahydroptérine, ou la sépiaptérine ou un mélange de celles-ci.

7. Composition pharmaceutique selon l'une quelconque des revendications 1 à 6, laquelle composition est appropriée pour une administration par voie orale, entérale, parentérale ou topique.

8. Composition pharmaceutique selon la revendication 7, laquelle composition comprend en outre un agent édulcorant, un agent aromatisant ou un mélange de ceux-ci.
